# FASCICULE DE BREVET EUROPEEN

(11) **EP 2 926 865 B1**
(45) Date de publication et mention de la délivrance du brevet: **09.08.2017**
(21) Numéro de dépôt: 15161124.1
(22) Date de dépôt: 26.03.2015
(51) Int. Cl.: A61N 1/375, H05K 5/00

(54) **PROCEDE DE REALISATION D'UN BOITIER HERMETIQUE DESTINE A L'ENCAPSULATION D'UN DISPOSITIF IMPLANTABLE ET BOITIER CORRESPONDANT**
HERSTELLUNGSVERFAHREN EINES HERMETISCH VERSCHLOSSENEN GEHÄUSES ZUM EINKAPSELN EINER IMPLANTIERBAREN VORRICHTUNG, UND ENTSPRECHENDES GEHÄUSE
METHOD FOR MANUFACTURING A SEALED HOUSING FOR ENCAPSULATING AN IMPLANTABLE DEVICE AND CORRESPONDING HOUSING

(30) Priorité: 31.03.2014 FR 1452790
(43) Date de publication de la demande: 07.10.2015
(73) Titulaire: COMMISSARIAT À L'ÉNERGIE ATOMIQUE ET AUX ÉNERGIES ALTERNATIVES, 75015 Paris (FR)
(72) Inventeur: Perraud, Simon, 83150 BANDOL (FR); Karst, Nicolas, 57600 FOLKLING (FR)
(74) Mandataire: Gevers & Orès

(56) Documents cités:
- EP-A1- 1 445 798
- EP-A2- 0 266 210
- WO-A1-2006/097842
- WO-A1-2013/099167
- WO-A1-2013/137214
- WO-A2-2012/015756
- US-A- 5 750 926

## Description

L'invention concerne un procédé de réalisation d'un boîtier hermétique notamment destiné à l'encapsulation d'un dispositif et plus particulièrement d'un dispositif médical implantable.

L'invention concerne également un tel boîtier hermétique.

Les dispositifs biomédicaux implantables, tels que les stimulateurs cardiaques, les défibrillateurs cardiaques, les moniteurs cardiaques, les pompes, les capteurs biomédicaux ou les dispositifs de neurostimulation, sont composés d'une batterie et d'un ensemble de composants électroniques encapsulés dans un boîtier métallique (généralement en titane) biocompatible.

En plus du caractère biocompatible, le boîtier assurant l'encapsulation des différents composants du dispositif doit être hermétique afin d'éviter tout contact entre les composants et les tissus ou fluides biologiques.

De façon générale, il est souhaitable que le volume occupé par ces dispositifs soit réduit. Dans le cas des neurostimulateurs, ceci permet d'adresser de nouvelles zones d'implantation et de s'approcher au plus près des zones à stimuler électriquement.

Pour ce faire, différentes solutions d'encapsulation hermétique et biocompatible ont pu être proposées dans la littérature comme alternative au boîtier en titane classiquement utilisé.

Ainsi, le brevet US- 5 750 926 de Schulman et al. décrit un boîtier assurant l'encapsulation des différents composants nécessaires au bon fonctionnement d'un neurostimulateur. Il est obtenu en fixant un couvercle métallique à un substrat isolant. Deux étapes sont nécessaires pour assurer l'herméticité du boîtier : la première étape prévoit la formation d'un premier joint hermétique entre un cadre métallique et le substrat isolant, généralement par brasage. La deuxième étape permet de réaliser un deuxième joint hermétique entre le cadre métallique et le couvercle métallique par soudage localisé et notamment par soudage laser.

Dans ce boîtier, il est difficile d'utiliser un cadre métallique fin, par exemple dont l'épaisseur est inférieure à 1 mm.

En effet, si un cadre métallique fin est utilisé, alors au moins deux problèmes peuvent survenir lors de la mise en oeuvre du procédé de fabrication :
- Lors de la formation du premier joint hermétique par brasage, des éléments chimiques contenus dans le joint de brasage diffusent à travers l'épaisseur du cadre métallique, ce qui résulte en une accroche indésirable du cadre métallique sur un porte-échantillon qui le maintient sur le substrat.
- Lors de la formation du deuxième joint hermétique par soudage au faisceau laser, le premier joint hermétique peut être dégradé thermiquement.

L'objet de l'invention est de réduire encore l'épaisseur des boîtiers d'encapsulation, sans compromettre leur tenue mécanique, notamment pour obtenir des boîtiers mécaniquement flexibles.

En effet, de tels boîtiers fins et flexibles permettent d'améliorer sensiblement le confort du patient et d'envisager une implantation dans des zones du corps humain difficilement accessibles à des dispositifs conventionnels. Dans le cas de neurostimulateurs implantables, ils permettent une implantation au plus près de la zone à stimuler électriquement, ce qui réduit les risques liés à la rupture des câbles d'extension et des sondes-électrodes.

Ainsi, l'invention concerne un procédé de réalisation d'un boîtier hermétique comprenant les étapes suivantes :
a) fournir un substrat en céramique,
b) fournir un cadre métallique et le mettre en regard dudit substrat,
c) former un premier joint hermétique à l'interface entre ledit substrat et ledit cadre métallique, pour les assembler et former un ensemble,
d) superposer audit ensemble un couvercle,
e) former un deuxième joint hermétique entre la face du cadre métallique opposée à ladite interface et le couvercle pour obtenir ledit boîtier.

Selon l'invention, lors de l'étape c), le premier joint hermétique est formé sur une portion de l'interface et le procédé comporte, avant l'étape c), une étape supplémentaire consistant à disposer un cadre en céramique sur la face du cadre métallique opposée à ladite interface, de façon à recouvrir partiellement cette face, la surface de projection dudit cadre en céramique dans un plan de projection recouvrant la surface de projection dudit premier joint dans ce même plan de projection.

De façon préférée, le premier joint hermétique formé lors de l'étape c) est un joint de brasage.

Dans une première variante, lors de l'étape a), peut être alors fourni un substrat dans lequel est formé un évidement, le joint de brasage étant au moins partiellement intégré dans ledit évidement.

Dans une deuxième variante, lors de l'étape b), peut être fourni un cadre métallique dans lequel est formé un évidement, le joint de brasage étant au moins partiellement intégré dans ledit évidement.

L'invention concerne également un procédé d'encapsulation d'un dispositif consistant à mettre en oeuvre le procédé de réalisation d'un boîtier hermétique selon l'invention et à monter au moins un composant dudit dispositif à encapsuler sur le substrat, après l'étape c).

L'invention concerne également un boîtier hermétique comprenant :
- un substrat en céramique,
- un cadre métallique relié hermétiquement au substrat par un premier joint situé sur une portion de l'interface entre ledit substrat et ledit cadre et
- un couvercle relié hermétiquement audit cadre métallique par un deuxième joint hermétique, de façon à définir une cavité,
caractérisé en ce qu'un cadre en céramique est relié audit cadre métallique sur sa face opposée à ladite interface, de façon à recouvrir partiellement ladite face du cadre, la surface de projection dudit cadre en céramique dans un plan de projection recouvrant la surface de projection dudit premier joint dans ce même plan de projection.

Dans une première variante de réalisation du boîtier, le cadre en céramique est situé à l'intérieur de la cavité.

Dans une deuxième variante de réalisation du boîtier, le cadre en céramique est situé à l'extérieur de la cavité.

Le premier joint hermétique est de préférence un joint de brasage.

Dans ce cas, ledit substrat et/ou ledit cadre métallique comporte(nt) avantageusement un évidement dans lequel est au moins partiellement intégré ledit joint de brasage.

L'invention sera mieux comprise et d'autres buts, avantages et caractéristiques de celle-ci apparaîtront plus clairement à la lecture de la description qui suit et qui est faite au regard des dessins annexés, sur lesquels :
- la figure 1 comprend les figures 1A à 1D qui sont des vues en coupe illustrant des étapes d'un procédé selon l'état de la technique permettant d'obtenir un boîtier hermétique,
- la figure 2 comprend les figures 2A à 2E et illustre les différentes étapes d'un procédé selon l'invention permettant d'obtenir un boîtier hermétique, et
- la figure 3 comprend les figures 3A et 3B qui sont des vues en coupe illustrant une variante de réalisation des étapes du procédé illustrées aux figures 2B et 2E.

Les éléments communs aux différentes figures seront désignés par les mêmes références.

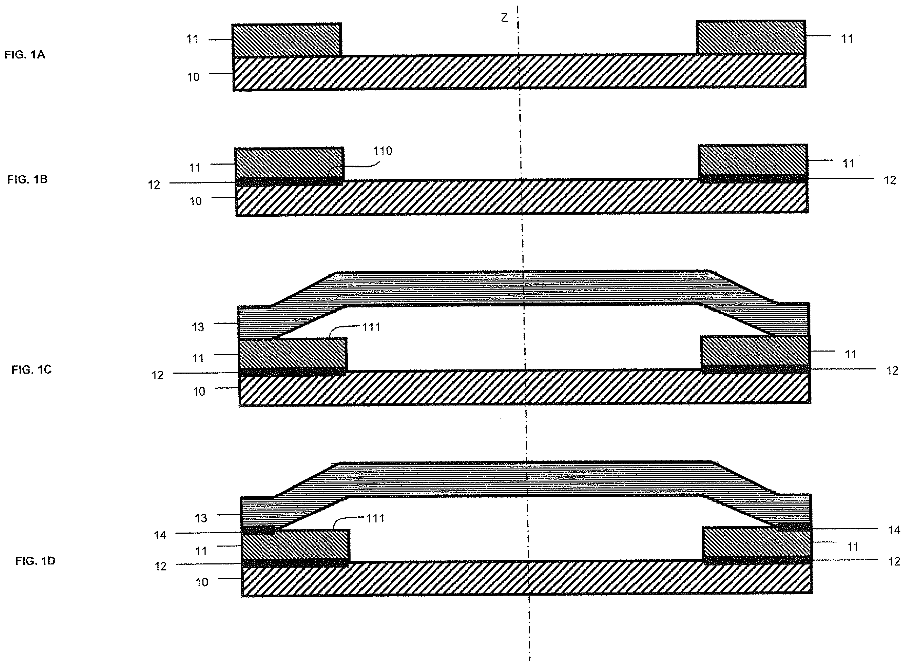

La figure 1A décrit les deux premières étapes d'un procédé selon l'état de la technique, dans lesquelles est fourni un substrat 10 en céramique, en regard duquel est placé un cadre métallique 11.

La figure 1B illustre une troisième étape du procédé dans laquelle est formé un premier joint hermétique 12 à l'interface entre le cadre métallique 11 et le substrat 10, ou encore entre une face inférieure 110 du cadre métallique et le substrat 10.

Ce premier joint hermétique 12 est formé par brasage et permet d'assembler le substrat et le cadre.

La figure 1C illustre une quatrième étape dans laquelle un couvercle métallique 13 est disposé sur le cadre métallique 11.

La figure 1D illustre une cinquième étape du procédé dans laquelle est formé un deuxième joint hermétique 14 à l'interface entre le cadre métallique 11 et le couvercle 13, ou encore entre une face supérieure 111 du cadre 11 et le couvercle 13.

Ce deuxième joint 14 est formé grâce à un procédé de soudage comportant un apport localisé de chaleur, comme un soudage au faisceau laser.

En pratique, avec un tel procédé, il s'avère difficile de réduire l'épaisseur du cadre métallique à une épaisseur inférieure à 1 mm.

En effet, en dessous de cette valeur, deux problèmes peuvent survenir.

Tout d'abord, lors de la formation du premier joint hermétique 12 par brasage, des éléments chimiques contenus dans le joint de brasage 12 diffusent à travers l'épaisseur du cadre métallique 11, ce qui résulte en une accroche indésirable de la face supérieure 111 du cadre métallique 11 sur le porte-échantillon. En effet, ce dernier (non représenté à la figure 1) est utilisé pour appliquer une pression sur la face supérieure 111 du cadre métallique 11, lors de la formation du premier joint hermétique 12.

Par exemple, dans le cas où le cadre métallique 11 est en titane et où le premier joint hermétique 12 est constitué de titane et de nickel, lors du recuit de brasage, le nickel diffuse à travers l'épaisseur du cadre métallique 11 et contribue à former une zone d'accroche indésirable entre la face supérieure 111 du cadre métallique 11 et le porte-échantillon.

De plus, lors de la formation du deuxième joint hermétique 14 par soudage au faisceau laser, le premier joint hermétique 12 peut être dégradé thermiquement.

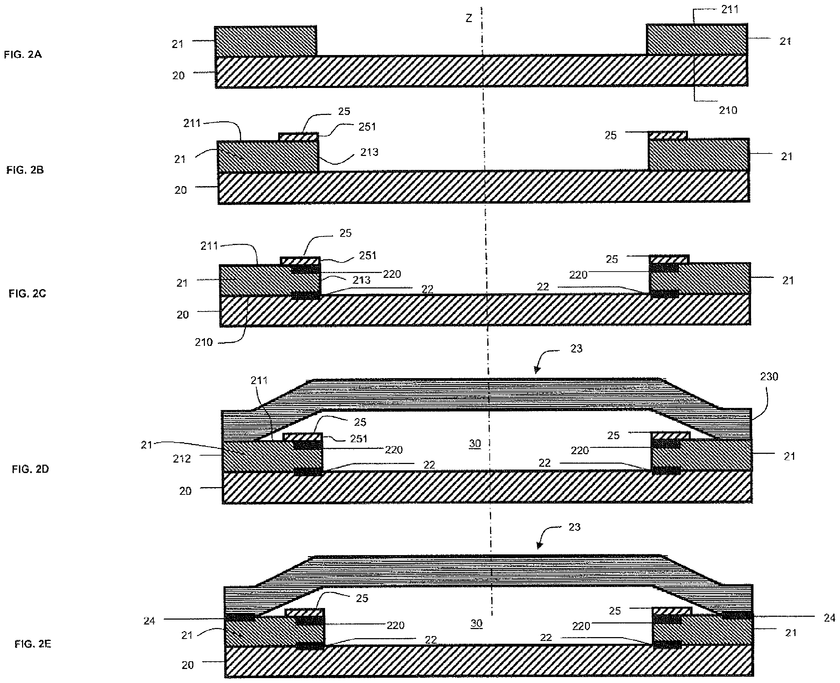

Il va maintenant être fait référence à la figure 2 qui illustre un procédé de fabrication d'un boîtier hermétique selon l'invention.

La figure 2A illustre les deux premières étapes du procédé qui sont identiques aux deux premières étapes du procédé selon l'état de la technique.

Elles consistent à fournir un substrat 20 en céramique et un cadre métallique 21 et à mettre ce dernier en regard du substrat 20.

Le substrat en céramique 20 peut être en alumine, zircone, zircone stabilisée à l'oxyde d'yttrium, ou zircone stabilisée à l'oxyde de cérium.

Avantageusement, le substrat en céramique 20 présente une surface comprise entre 10 mm² et 100 cm² et une épaisseur comprise entre 10 µm et 1 mm et, de préférence, entre 10 µm et 100 µm.

Le cadre métallique 21 peut être en titane ou en alliage de titane. Avantageusement, le cadre métallique 21 présente une largeur comprise entre 1 mm et 1 cm et une épaisseur comprise entre 10 µm et 1 mm et, de préférence, entre 10 µm et 100 µm.

La figure 2B illustre une autre étape du procédé dans laquelle est disposé un cadre en céramique 25 sur la face supérieure 211 du cadre 21, face qui est opposée à l'interface entre le cadre métallique 25 et le substrat 20.

Par ailleurs, ce cadre en céramique 25 ne recouvre que partiellement la face supérieure 211 du cadre 21.

Dans l'exemple illustré sur la figure 2C, le cadre 25 est situé du côté de la face intérieure 213 du cadre métallique 21.

De façon plus précise, le bord intérieur 251 du cadre 25 est, dans cet exemple, situé sensiblement à l'aplomb de la face intérieure 213 du cadre métallique 21.

Le cadre en céramique 25 peut être en alumine, zircone, zircone stabilisée à l'oxyde d'yttrium ou zircone stabilisée à l'oxyde de cérium.

Avantageusement, le cadre en céramique 25 présente une largeur comprise entre 1 mm et 1 cm et une épaisseur comprise entre 10 µm et 1 mm et, de préférence, entre 10 µm et 100 µm.

La figure 2C illustre l'étape suivante dans laquelle est formé un premier joint hermétique 22 à l'interface entre le substrat 20 et le cadre métallique 21, c'est-à-dire entre la face inférieure 210 du cadre 21, opposée à la face supérieure 211, et le substrat 20.

Ce joint 22 est formé sur une portion de l'interface qui est en regard du cadre en céramique 25. Cette portion est donc aussi située vers l'intérieur du boîtier en cours de réalisation.

Ainsi, en considérant un plan de projection commun au cadre 25 et au joint 22, par exemple le plan du substrat 20, la surface de projection du cadre 25 dans ce plan de projection est au moins égale à la surface de projection du premier joint hermétique 22 dans ce même plan de projection. De plus, la position relative du cadre 25 et du joint 22 est telle que la surface de projection du cadre 25 recouvre intégralement la surface de projection du joint 22.

En aucun cas, le joint 22 est formé sur la totalité de l'interface.

De manière générale, le pourcentage de surface de l'interface occupée par le joint 22 est compris entre 20 et 80% pour permettre à la fois un assemblage efficace et la mise en place d'un couvercle.

Ce premier joint hermétique 22 peut être constitué de titane et de nickel.

Lors de la formation du premier joint hermétique 22, une zone d'accroche 220 est simultanément formée entre la face supérieure 211 du cadre métallique 21 et le cadre en céramique 25. En effet, ceci résulte de la diffusion des éléments chimiques contenus dans le joint de brasage 22 à travers l'épaisseur du cadre métallique 21.

Le cadre en céramique 25 quant à lui n'accroche pas sur le porte-échantillon (non représenté à la figure 2) qui est utilisé pour appliquer une pression sur le cadre en céramique 25, et donc indirectement sur la face supérieure 211 du cadre métallique 21, lors de la formation du premier joint hermétique 22.

Par exemple, dans le cas où le cadre métallique 21 est en titane et où le premier joint hermétique 22 est constitué de titane et de nickel, lors du recuit de brasage, le nickel diffuse à travers l'épaisseur du cadre métallique 21 et contribue à former la zone d'accroche 220 entre la face supérieure 211 du cadre métallique 21 et le cadre en céramique 25. Au début du recuit de brasage, le nickel est uniquement présent au niveau du premier joint hermétique 22. Cependant, à l'issue du recuit de brasage, le nickel se retrouve également au niveau de la zone d'accroche 220, ainsi que dans toute la partie du cadre métallique 21 comprise entre le premier joint hermétique 22 et la zone d'accroche 220.

Ainsi, à l'issue du recuit de brasage, trois zones (premier joint hermétique 22, zone d'accroche 220 et partie du cadre métallique 21 située entre le joint 22 et la zone d'accroche 220) présentent une composition chimique similaire, ces trois zones contenant du titane et du nickel.

La figure 2D illustre l'étape suivante du procédé dans laquelle un couvercle métallique 23 est disposé sur la face supérieure 211 du cadre métallique 21.

Dans le mode de réalisation illustré à la figure 2D, le bord extérieur 230 du couvercle 23 est sensiblement à l'aplomb de la face extérieure 212 du cadre métallique 21, face opposée à la surface intérieure 213.

Ainsi, le cadre en céramique 25 est situé à l'intérieur de la cavité 30 du boîtier en cours de formation.

Le couvercle métallique 23 peut être en titane ou en alliage de titane.

Avantageusement, le couvercle métallique 23 présente une épaisseur comprise entre 10 µm et 1 mm et, de préférence, entre 10 µm et 100 µm.

Dans une dernière étape de réalisation du procédé illustrée à la figure 2E, est formé un deuxième joint hermétique 24 entre la face supérieure 211 du cadre métallique 21 et le couvercle 23.

Ce joint 24 est formé par un procédé de soudage réalisé avec un apport localisé de chaleur, comme un soudage au faisceau laser.

Lors de la formation du deuxième joint hermétique 24, le risque de dégrader thermiquement le premier joint hermétique 22 est très faible, car le deuxième joint hermétique 24 est décalé latéralement par rapport au premier joint hermétique 22, le premier joint 22 étant situé à l'intérieur de la cavité 30 et le deuxième joint 24 en périphérie de la cavité.

Ainsi, les deux joints 22 et 24 sont décalés à la fois dans un plan de projection commun, parallèle au substrat 20, et dans un autre plan de projection commun, perpendiculaire au substrat 20.

Le procédé illustré aux figures 2A à 2E permet de réaliser un boîtier hermétique fin en intégrant un cadre métallique dont l'épaisseur peut être réduite, sans impact négatif sur le procédé de réalisation. En particulier, la présence du cadre en céramique permet d'éviter toute accroche d'un porte-échantillon sur le cadre métallique, malgré la faible épaisseur de ce cadre.

De plus, le décalage latéral entre les deux joints hermétiques permet d'éviter toute dégradation du premier joint hermétique lors de la formation du deuxième joint.

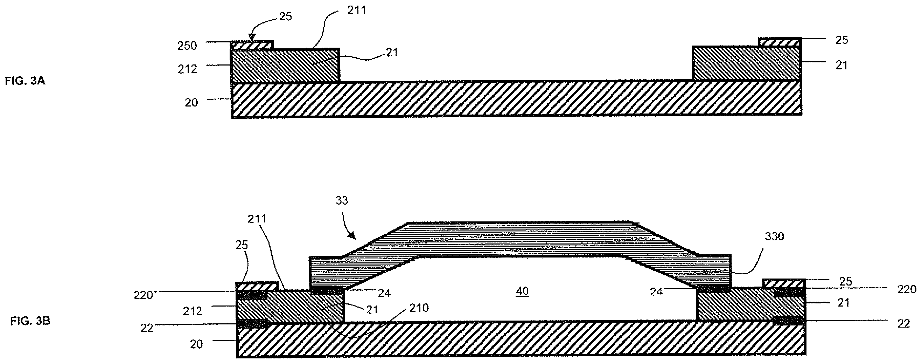

Il est maintenant fait référence à la figure 3 qui illustre une variante de réalisation du procédé illustré à la figure 2.

Comme illustré à la figure 3A, cette variante de réalisation consiste à disposer le cadre en céramique 25 du côté extérieur du cadre métallique 21 de façon à recouvrir partiellement la face supérieure 211 du cadre 21.

Ainsi, dans l'exemple illustré à la figure 3A, le bord extérieur 250 du cadre en céramique 25, opposé au bord intérieur 251, est situé sensiblement à l'aplomb de la face extérieure 212 du cadre métallique 21, opposée à la face intérieure 213.

La formation du premier joint hermétique 22 à l'interface entre le cadre métallique 21 et le substrat 20 est réalisé comme décrit précédemment au regard de la figure 2C et ne sera pas décrit plus en détail.

Cette étape permet d'obtenir non seulement le joint hermétique 22 entre la face inférieure 210 du cadre 21 et le substrat 20 mais également une zone d'accroche 220 entre la face supérieure 211 du cadre 21 et le cadre en céramique 25.

Comme dans le procédé illustré à la figure 2, la surface de projection du cadre en céramique 25 dans un plan de projection est au moins égale à la surface de projection du joint 22 dans ce même plan. Ce plan de projection commun peut notamment comprendre le joint 22 et correspondre au plan du substrat 20.

De plus, la position relative du cadre en céramique 25 et du joint 22 est telle que la surface de projection du cadre 25 recouvre totalement la surface de projection du joint 22, dans ce plan de projection commun.

Ainsi, le cadre 25 évite toute accroche d'un porte-échantillon sur le cadre métallique 21.

Le couvercle 33 est ensuite superposé à l'ensemble obtenu.

La figure 3B montre que, dans cette variante de réalisation du procédé, le bord extérieur 330 du couvercle 33 est situé vers l'intérieur du boîtier en cours de réalisation par rapport au cadre en céramique 25.

La dernière étape du procédé consiste à former le deuxième joint hermétique 24, comme cela a été décrit en référence à la figure 2E.

La figure 3B montre le boîtier ainsi obtenu.

Contrairement au boîtier illustré à la figure 2E, le cadre en céramique 25 est ici situé à l'extérieur de la cavité 40 formée par le boîtier.

Cette deuxième variante de réalisation du procédé présente les mêmes avantages que ceux décrits en référence au procédé décrit en référence à la figure 2.

Là encore, le décalage latéral entre les deux joints hermétiques permet d'éviter toute dégradation du premier joint hermétique 22 lors de la formation du deuxième joint 24.

En effet, le premier joint 22 est situé à l'extérieur de la cavité 40 tandis que le deuxième joint est situé en périphérie de cette cavité.

Une autre variante du procédé selon l'invention consiste à réaliser un évidement dans le substrat en céramique 20, évidement dans lequel est formé le joint de brasage 22, le joint étant au moins partiellement intégré dans cet évidement.

Une autre variante du procédé consiste à prévoir un tel évidement dans le cadre métallique 21.

Il est également possible de combiner ces deux variantes en prévoyant un évidement à la fois dans le substrat en céramique 20 et dans le cadre métallique 21.

Dans les trois cas, la présence de cet évidement permet de réduire encore l'épaisseur du boîtier hermétique obtenu par le procédé selon l'invention, puisque le joint de brasage se situe en partie dans l'évidement réalisé dans le substrat et/ou le couvercle. En effet, l'impact de l'épaisseur du joint de brasage sur l'épaisseur du boîtier est ainsi réduit.

Les signes de référence insérés après les caractéristiques techniques figurant dans les revendications ont pour seul but de faciliter la compréhension de ces dernières et ne sauraient en limiter la portée.

## Revendications

1. Procédé de réalisation d'un boîtier hermétique pour un dispositif implantable comprenant les étapes suivantes :
a) fournir un substrat (20) en céramique,
b) fournir un cadre métallique (21) et le mettre en regard dudit substrat (20),
c) former un premier joint hermétique (22) à l'interface entre ledit substrat (20) et ledit cadre métallique (21), pour les assembler et former un ensemble,
d) superposer audit ensemble un couvercle (23, 33),
e) former un deuxième joint hermétique (24) entre une face supérieure (211) du cadre métallique (21) opposée à ladite interface et le couvercle (23, 33) pour obtenir ledit boîtier,
**caractérisé en ce que**, lors de l'étape c), le premier joint hermétique (22) est formé sur une portion de ladite interface et **en ce qu'**avant l'étape c), le procédé comporte une étape supplémentaire consistant à disposer un cadre en céramique (25) sur la face supérieure (211) du cadre métallique, de façon à recouvrir partiellement ladite face du cadre métallique, la surface de projection dudit cadre en céramique (25) dans un plan de projection commun audit cadre en céramique et audit premier joint hermétique, et recouvrant la surface de projection dudit premier joint dans ce même plan de projection.

2. Procédé selon la revendication 1, **caractérisé en ce que** le premier joint hermétique (22) formé lors de l'étape c) est un joint de brasage.

3. Procédé selon la revendication 2, **caractérisé en ce que**, lors de l'étape a), est fourni un substrat dans lequel est formé un évidement, le joint de brasage étant au moins partiellement intégré dans ledit évidement.

4. Procédé selon la revendication 2 ou 3, **caractérisé en ce que**, lors de l'étape b), est fourni un cadre métallique dans lequel est formé un évidement, le joint de brasage étant au moins partiellement intégré dans ledit évidement.

5. Procédé d'encapsulation d'un dispositif consistant à mettre en oeuvre le procédé selon l'une des revendications 1 à 4 et à monter au moins un composant dudit dispositif à encapsuler sur ledit substrat (20), après l'étape c).

6. Boîtier hermétiques pour un dispositif implantable comprenant :
- un substrat (20) en céramique,
- un cadre (21) métallique relié hermétiquement au substrat par un premier joint (22) situé sur une portion de l'interface entre ledit substrat (20) et ledit cadre (21) et
- un couvercle (23, 33) relié hermétiquement audit cadre (21) métallique par un deuxième joint hermétique (24), de façon à définir une cavité (30, 40), **caractérisé en ce qu'**un cadre (25) en céramique est relié audit cadre métallique (21) sur une face (211) opposée à ladite interface, de façon à recouvrir partiellement ladite face (211) du cadre, la surface de projection dudit cadre en céramique (25) dans un plan de projection commun audit cadre en céramique et audit premier joint hermétique, recouvrant la surface de projection dudit premier joint dans ce même plan de projection.

7. Boîtier selon la revendication 6, **caractérisé en ce que** le cadre (25) est situé à l'intérieur de la cavité (30).

8. Boîtier selon la revendication 6, **caractérisé en ce que** le cadre (25) en céramique est situé à l'extérieur de la cavité (40).

9. Boîtier selon l'une des revendications 6 à 8, **caractérisé en ce que** le premier joint hermétique (22) est un joint de brasage.

10. Boîtier selon l'une des revendications 6 à 9, **caractérisé en ce que** ledit substrat (20) et/ou ledit cadre métallique (21) comporte un évidement dans lequel est au moins partiellement intégré le premier joint hermétique.

## Patentansprüche

1. Verfahren zur Herstellung eines hermetisch verschlossenen Gehäuses für eine implantierbare Vorrichtung, das folgende Schritte umfasst:
a) ein Substrat (20) aus Keramik bereitstellen,
b) einen Metallrahmen (21) bereitstellen und ihn dem Substrat (20) gegenüber anordnen,
c) eine erste hermetische Dichtung (22) an der Schnittstelle zwischen dem Substrat (20) und dem Metallrahmen (21) bilden, um sie zu verbinden und eine Einheit zu bilden,
d) der Einheit einen Deckel (23, 33) aufsetzen,
e) eine zweite hermetische Dichtung (24) zwischen einer Oberseite (211) des Metallrahmens (21) entgegengesetzt zur Schnittstelle und dem Deckel (23, 33) bilden, um das Gehäuse zu erzielen,
**dadurch gekennzeichnet, dass** beim Schritt c) die erste hermetische Dichtung (22) auf einem Abschnitt der Schnittstelle gebildet wird, und dadurch, dass das Verfahren vor dem Schritt c) einen zusätzlichen Schritt umfasst, der darin besteht, einen Keramikrahmen (25) auf der Oberseite (211) des Metallrahmen anzuordnen, so dass die Fläche des Metallrahmens und die Projektionsfläche des Keramikrahmens (25) in einer mit dem Keramikrahmen und der ersten hermetischen Dichtung gemeinsamen Projektionsebene abgedeckt werden, und die Projektionsfläche der ersten Dichtung in dieser gleichen Projektionsebene abgedeckt wird.

2. Verfahren nach Anspruch 1, **dadurch gekennzeichnet, dass** die erste beim Schritt c) gebildete hermetische Dichtung (22) eine Lötnaht ist.

3. Verfahren nach Anspruch 2, **dadurch gekennzeichnet, dass** beim Schritt a) ein Substrat bereitgestellt wird, in dem eine Ausnehmung ausgebildet ist, wobei die Lötnaht zumindest teilweise in die Ausnehmung eingebunden ist.

4. Verfahren nach Anspruch 2 oder 3, **dadurch gekennzeichnet, dass** beim Schritt b) ein Metallrahmen bereitgestellt wird, in dem eine Ausnehmung ausgebildet ist, wobei die Lötnaht zumindest teilweise in die Ausnehmung eingebunden ist.

5. Verfahren zum Einkapseln einer Vorrichtung, das darin besteht, das Verfahren nach einem der Ansprüche 1 bis 4 einzusetzen und nach dem Schritt c) zumindest eine Komponente der einzukapselnden Vorrichtung auf das Substrat (20) zu montieren.

6. Hermetisch verschlossenes Gehäuse für eine implantierbare Vorrichtung, das Folgendes umfasst:
- ein Substrat (20) aus Keramik,
- einen Metallrahmen (21), der durch eine erste Dichtung (22) hermetisch mit dem Substrat verbunden ist, die sich auf einem Abschnitt der Schnittstelle zwischen dem Substrat (20) und dem Rahmen (21) befindet, und
- einen Deckel (23, 33), der durch eine zweite Dichtung (24) hermetisch mit dem Metallrahmen (21) verbunden ist, damit eine Aussparung (30, 40) definiert wird,
**dadurch gekennzeichnet, dass** ein Keramikrahmen (25) mit dem Metallrahmen (21) auf einer Fläche (211) entgegengesetzt zur Schnittstelle verbunden ist, so dass die Fläche (211) des Rahmens und die Projektionsfläche des Keramikrahmens (25) in einer mit dem Keramikrahmen und der ersten hermetischen Dichtung gemeinsamen Projektionsebene abgedeckt werden, und die Projektionsfläche der ersten Dichtung in dieser gleichen Projektionsebene abgedeckt wird.

7. Gehäuse nach Anspruch 6, **dadurch gekennzeichnet, dass** sich der Rahmen (25) im Innern der Aussparung (30) befindet.

8. Gehäuse nach Anspruch 6, **dadurch gekennzeichnet, dass** sich der Keramikrahmen (25) außerhalb der Aussparung (40) befindet.

9. Gehäuse nach einem beliebigen der vorstehenden Ansprüche 6 bis 8, **dadurch gekennzeichnet, dass** die erste hermetische Dichtung (22) eine Lötnaht ist.

10. Gehäuse nach einem beliebigen der vorstehenden Ansprüche 6 bis 9, **dadurch gekennzeichnet, dass** das Substrat (20) und/oder der Metallrahmen (21) eine Ausnehmung umfasst, in die die erste hermetische Dichtung zumindest teilweise eingebunden ist.

## Claims

1. Method for producing a hermetically sealed casing for an implantable device, comprising the following steps:
a) supplying a ceramic substrate (20),
b) supplying a metal surround (21) and placing it facing the said substrate (20),
c) forming a first hermetically sealed joint (22) at the interface between the said substrate (20) and the said metal surround (21), in order to assemble them and form an assembly,
d) superposing a cover (23, 33) on the said assembly,
e) forming a second hermetically sealed joint (24) between an upper face (211) of the metal surround (21) which is the opposite face to the said interface, and the cover (23, 33), in order to obtain the said casing,
**characterized in that**, during step c), the first hermetically sealed joint (22) is formed on a portion of the said interface and **in that** prior to step c), the method involves an additional step consisting in placing a ceramic surround (25) on the upper face (211) of the metal surround so as to partially cover the said face of the metal surround, the projected surface of the said ceramic surround (25) in a plane of projection covering the projected surface of the said first joint in this same plane of projection.

2. Method according to Claim 1, **characterized in that** the first hermetically sealed joint (22) formed during step c) is a brazed joint.

3. Method according to Claim 2, **characterized in that**, during step a), a substrate in which a recess is formed is supplied, the brazed joint being incorporated at least partially into the said recess.

4. Method according to Claim 2 or 3, **characterized in that**, during step b), a metal surround in which a recess is formed is supplied, the brazed joint being at least partially incorporated into the said recess.

5. Method of encapsulating a device consisting in implementing the method according to one of Claims 1 to 4 and in mounting at least one component of the said device that is to be encapsulated on the said substrate (20), after step c).

6. Hermetically sealed casing for an implantable device comprising:
- a ceramic substrate (20),
- a metal surround (21) hermetically connected to the substrate by a first joint (22) situated on a portion of the interface between the said substrate (20) and the said surround (21), and
- a cover (23, 33) hermetically connected to the said metal surround (21) by a second hermetically sealed joint (24), so as to define a cavity (30, 40),
**characterized in that** a ceramic surround (25) is connected to the said metal surround (21) on an opposite face (211) to the said interface, so as to partially cover the said face (211) of the surround, the projected surface of the said ceramic surround (25) in a plane of projection covering the projected surface of the said first joint in this same plane of projection.

7. Casing according to Claim 6, **characterized in that** the surround (25) is situated on the inside of the cavity (30).

8. Casing according to Claim 6, **characterized in that** the ceramic surround (25) is situated on the outside of the cavity (40).

9. Casing according to one of Claims 6 to 8, **characterized in that** the first hermetically sealed joint (22) is a brazed joint.

10. Casing according to one of Claims 6 to 9, **characterized in that** the said substrate (20) and/or the said metal surround (21) comprises a recess into which the first hermetically sealed joint is at least partially incorporated.
